# EUROPEAN PATENT APPLICATION

(11) **EP 4 650 430 A1**
(43) Date of publication of application: **19.11.2025**
(21) Application number: 24741577.1
(22) Date of filing: 12.01.2024
(51) Int. Cl.: C12M 1/00, F16L 11/06

(54) **TUBE AND CELL CULTURE DEVICE**

(30) Priority: 12.01.2023 JP 2023003203
(71) Applicant: TOKYO OHKA KOGYO CO., LTD., Kawasaki-shi, Kanagawa 211 0012 (JP); PUBLIC UNIVERSITY CORPORATION NAGOYA CITY UNIVERSITY, Nagoya-shi, Aichi 467-8601 (JP)
(72) Inventor: KUMAZAWA Hirotsugu, Kawasaki-shi, Kanagawa 211-0012 (JP); YOSHIOKA Takahiro, Kawasaki-shi, Kanagawa 211-0012 (JP); MATSUNAGA Tamihide, Nagoya-shi, Aichi 467-8603 (JP); IWAO Takahiro, Nagoya-shi, Aichi 467-8603 (JP)
(74) Representative: Plasseraud IP
(86) International application number: PCT/JP2024/000533
(87) International publication number: WO 2024/150805

(57) **Abstract**

One aspect of the present invention is a piping tube (100) in a cell culture device, which is characterized by including a tubular body (10) made of a fluororesin, in which the tubular body (10) is made of a fluororesin having a constitutional unit represented by General Formula (f1-1) and a constitutional unit represented by General Formula (f2), a hardness of the tubular body (10) is 60 to 75, and a thickness (L_{T}) of the tube (100) is 0.1 to 1 mm. The piping tube (100) has low drug sorption properties and also has favorable moldability into a tube shape and favorable adaptability to pump connection and the like. In the general formula, R¹ to R⁷ are each independently a fluorine atom or a hydrogen atom. R⁸ is a trifluoromethyl group, a methyl group, or a group represented by -O-Rf. Rf is a fluoroalkyl group having 1 to 15 carbon atoms. However, at least one of R¹ to R⁸ contains a fluorine atom.

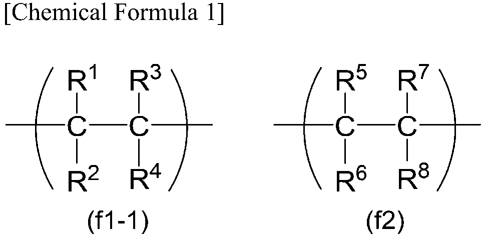

## Description

### TECHNICAL FIELD

The present invention relates to a tube and a cell culture device.

Priority is claimed on Japanese Patent Application No. 2023-003203, filed on January 12, 2023, the content of which is incorporated herein by reference.

### BACKGROUND ART

A cell culture device in which cells are cultured by allowing a fluid for cell culture to flow through a flow channel is known. This cell culture device is equipped with a liquid feeding mechanism (a liquid feeding tank, a liquid feeding tube, a feeding pump, and the like) for supplying a chemical liquid to a flow channel of a cell culture chip; a liquid drainage mechanism (a liquid drainage tank, a liquid drainage tube, a discharge pump, and the like) for discharging the chemical liquid from the flow channel of the cell culture chip; a temperature maintenance mechanism (a thermostat, and the like) for maintaining the culture temperature of the cell culture chip; a cleaning mechanism (a cleaning liquid tank, a tube, a pump, and the like) for cleaning the flow channel of the cell culture chip; and the like.

For example, the liquid feeding mechanism included in the cell culture device uses a liquid feeding device that supplies a chemical liquid containing a drug such as a cytosolic substrate to the cell culture chip. In such a system for feeding a chemical liquid, low sorption of the drug to the members used in the entire liquid feeding device is required. On the other hand, in the related art, a material having low drug sorption properties is used for a tube through which a chemical liquid flows.

Patent Document 1 proposes a fluid circuit device or a cell culture device, which is equipped with a block having a guide part that is attachable to and detachable from a pipe through which a chemical liquid fed from a pump flows, where the guide part includes at least one of a recessed part that guides the pipe along the surface of the block and a hole part that guides the pipe through the inside of the block.

In the device proposed in Patent Document 1, the block includes a plurality of sub-blocks that are attachably and detachably divided from each other, and a plurality of tubes are provided in correspondence to the plurality of sub-blocks.

The device is equipped with a plurality of tubes, which are each a pump connection tube connectable to a pump, a relay tube for relaying a pipe, a linking tube capable of linking the pump connection tube and the relay tube, and a tank connection tube connectable to a tank for storing a culture solution.

In this device, it is said that in a case where the block is molded from an ABS resin, the tube may be molded from polyether ether ketone (PEEK) or polytetrafluoroethylene (PTFE).

### Citation List

### Patent Document

[Patent Document 1] PCT International Publication No. WO2022/190627

### SUMMARY OF INVENTION

### Technical Problem

As described above, a material having low drug sorption properties is generally used for the tube constituting the liquid feeding device. However, particularly for a pump connection tube or a linking tube, the flexibility of the tube is further required in addition to the moldability into a tube shape.

However, as a general-purpose tube material, silicone rubber has high drug sorption properties, which causes a problem in the reliability of the cell culture test. In addition, in a case where PEEK or PTFE is molded into a tube shape, it cannot withstand the squeezing of the pump or the movement of the connecting part, which deteriorates the adaptability to the pump connection and the like.

The present invention has been made in consideration of the above circumstances, and an object of the present invention is to provide a tube that has low drug sorption properties and also has favorable moldability into a tube shape and favorable adaptability to pump connection and the like, and a cell culture device in which this tube is used.

### Solution to Problem

In order to achieve the above-described object, the present invention employs the following configurations.

That is, a first aspect of the present invention is a piping tube in a cell culture device, which is characterized by including a tubular body made of a fluororesin, in which the tubular body is made of a fluororesin having a constitutional unit (f1-1) represented by General Formula (f1-1) and a constitutional unit (f2) represented by General Formula (f2), a hardness of the tubular body is 60 to 75, and a thickness of the tube is 0.1 to 1 mm.

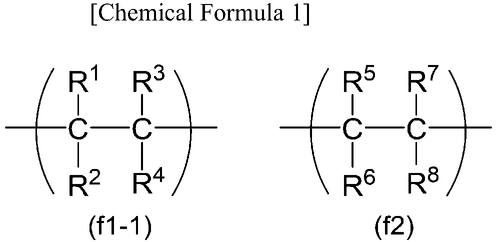

[In the formula, R¹ to R⁷ are each independently a fluorine atom or a hydrogen atom. R⁸ is a trifluoromethyl group, a methyl group, or a group represented by -O-Rf. Rf is a fluoroalkyl group having 1 to 15 carbon atoms. However, at least one of R¹ to R⁸ contains a fluorine atom.]

A second aspect of the present invention is a cell culture device, which is characterized in that the tube according to the first aspect is used for a pipe. Advantageous Effects of Invention

According to the present invention, it is possible to provide a tube that has low drug sorption properties and also has favorable moldability into a tube shape and favorable adaptability to pump connection and the like, and a cell culture device in which this tube is used.

### BRIEF DESCRIPTION OF DRAWINGS

[FIG. 1] A cross-sectional view showing one embodiment of a tube.
[FIG. 2] A schematic view of a fluid circuit device that is used in the evaluation of the examples.
[FIG. 3A] A graph showing a change in a residual rate of bufuralol in a cocktail substrate solution versus perfusion time.
[FIG. 3B] A graph showing a change in a residual rate of midazolam in a cocktail substrate solution versus perfusion time.

### DESCRIPTION OF EMBODIMENTS

### (Tube)

The tube according to the first aspect of the present invention can be used for a pipe in a cell culture device. The tube according to the present aspect is suitable as a pump connection tube connectable to a pump, and a linking tube capable of linking a relay a tube for relaying pipe to a pump connection tube, and it is more suitable as a pipe tube that is provided to a pump of a cell culture device. The tube according to the present aspect is particularly useful as a tube for a so-called tube pump.

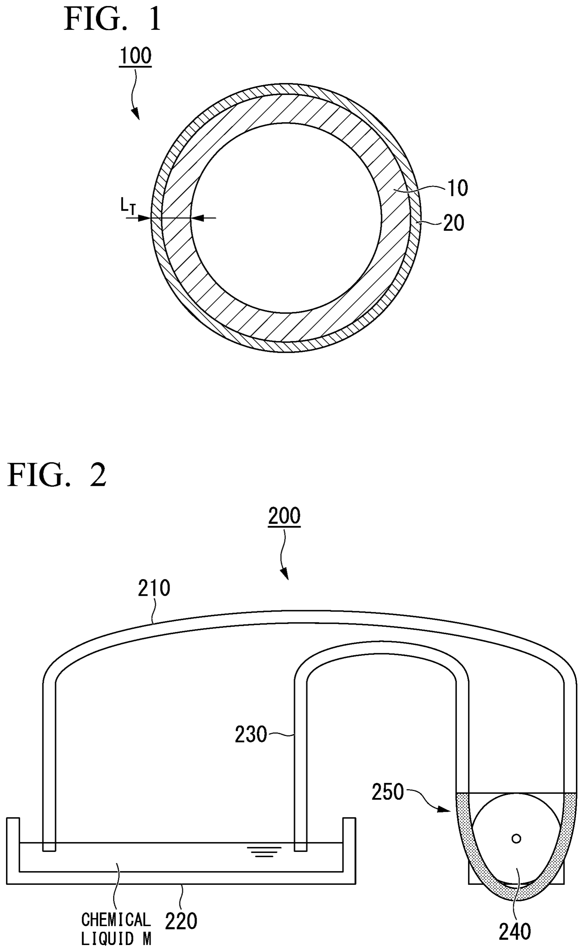

FIG. 1 is a cross-sectional view showing one embodiment of the tube according to the present aspect.

A tube 100 shown in FIG. 1 includes a tubular body 10 and an outer layer 20 that coats the outer surface of the tubular body 10.

For example, the outer diameter of the tube 100 is 0.7 to 4 mm, and the inner diameter of the tube 100 is 0.5 to 2 mm.

The thickness (L_{T}) of the tube 100 is 0.1 to 1 mm and preferably 0.2 to 0.7 mm. In a case where the thickness (L_{T}) is equal to or larger than the lower limit value of the above-described range, durability is improved, and in a case where the thickness (L_{T}) is equal to or less than the upper limit value of the above-described range, flexibility is maintained and thus the adaptability to pump connection and the like is improved.

The thickness (L_{T}) of the tube 100 is determined by (outer diameter - inner diameter)/2.

In the tube 100, the thickness of the outer layer 20 is, for example, 0.1 to 0.7 mm.

Examples of the materials of the outer layer 20 include a silicone resin, nitrile, urethane, and ethylene propylene.

### <Tubular body>

In the tube 100, the tubular body 10 is made of a fluororesin, and consists of a molded body of a fluororesin.

The thickness of the tubular body 10 is, for example, 0.1 to 0.5 mm.

In the present invention, the term "hardness of a tubular body" refers to a hardness of a molded body of a fluororesin.

The hardness of the tubular body, that is, the hardness of the molded body of the fluororesin, can be measured by a durometer hardness test, which is a test in accordance with the JIS standard (JIS K6253).

As a measuring method for the hardness of the tubular body, specifically, a method according to the following procedure is used.

Procedure (1): A plate-shaped test piece is prepared as a molded body of a fluororesin. The thickness of the test piece is 6.0 mm or more; the upper and lower surfaces of the test piece are planar and are set to be parallel to each other. The test piece should be measurable at a position where the distance from the press needle (measurement needle) to the end part of the test piece is 12.0 mm or more.

Procedure (2): A testing machine of which the pressurization plate, the press needle (measurement needle), the instruction mechanism, the spring force, and the like conform to the standards (JIS K6253 Type A durometer) is prepared.

Procedure (3): The hardness at five positions on the test piece (positions spaced apart from each other by 6.0 mm or more) is measured as follows. The test piece is placed on a flat surface, the press needle is brought into contact with one surface of the test piece perpendicularly, and the measured value is read 3 seconds after a pressurization of 1 kg is applied. The average value at the five positions in the test pieces is defined as the hardness of the molded body of the fluororesin (the hardness of the tubular body).

The hardness of the tubular body 10 is 60 to 75, and preferably 65 to 70.

In a case where the hardness of the tubular body 10 is equal to or larger than the lower limit value of the above-described range, the moldability into a tube shape is improved, and in a case where the hardness of the tubular body 10 is equal to or lower than the upper limit value of the above-described range, the adaptability to pump connection and the like is improved.

In the tube 100, the tubular body 10 is made of a fluororesin having a constitutional unit (f1-1) represented by General Formula (f1-1) and a constitutional unit (f2) represented by General Formula (f2). Since the inner surface of the tubular body 10 made of a fluororesin comes into contact with a chemical liquid, the drug sorption properties are deteriorated.

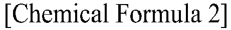

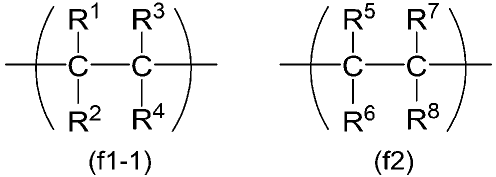

[In the formula, R¹ to R⁷ are each independently a fluorine atom or a hydrogen atom. R⁸ is a trifluoromethyl group, a methyl group, or a group represented by -O-Rf. Rf is a fluoroalkyl group having 1 to 15 carbon atoms. However, at least one of R¹ to R⁸ contains a fluorine atom.]

In Formula (f1-1), each of R¹ to R⁴ is preferably a fluorine atom.

In Formula (f2), it is preferable that R⁵ to R⁷ are all fluorine atoms or all hydrogen atoms.
- Rf in R⁸ in Formula (f2) may be a fluoroalkyl group having 10 to 15 carbon atoms or may be a perfluoroalkyl group having 10 to 15 carbon atoms. Examples thereof include -CH₂-(CF₂)₁₁-CF₃ and -(CF₂)₁₄-CF₃.

Specific examples of the constitutional unit (f1-1) are as follows.

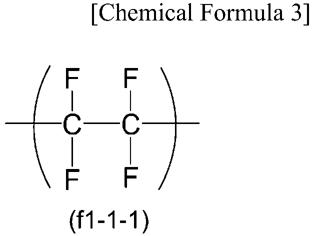

Specific examples of the constitutional unit (f2) are shown below.

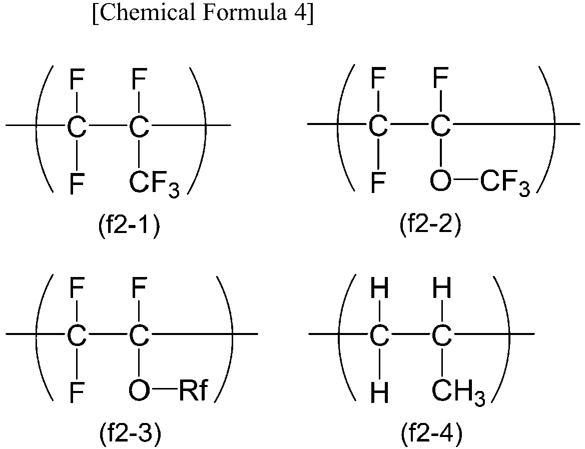

In the tube 100, the tubular body 10 may be made of a fluororesin having the constitutional unit (f1-1), the constitutional unit (f2), and a constitutional unit (f1-2) represented by General Formula (f1-2) (however, the constitutional unit (f1-2) has a structure different from a structure of the constitutional unit (f1-1)).

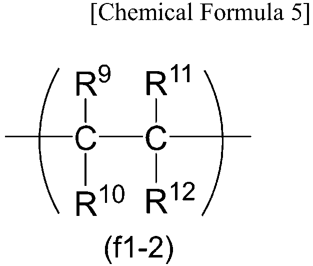

[In the formula, R⁹ to R¹² are each independently a fluorine atom or a hydrogen atom.]

In Formula (f1-2), it is preferable that R⁹ to R¹² are all hydrogen atoms.

Alternatively, it is preferable that R⁹ and R¹⁰ are each a hydrogen atom, and R¹¹ and R¹² are each a fluorine atom.

Specific examples of the constitutional unit (f1-2) are as follows.

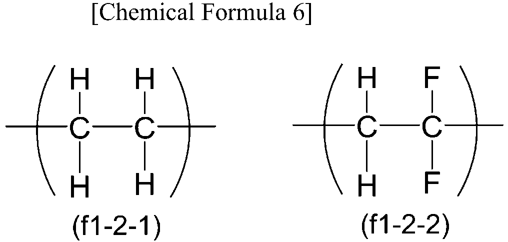

Preferred examples of the fluororesin constituting the tubular body 10 include each of fluororesins (F-1) to (F-6) which are represented by Chemical Formulae (F-1) to (F-6).

Fluororesin (F-1): A copolymer having a repeating structure of a constitutional unit (f1-1-1) and a constitutional unit (f2-2)
Fluororesin (F-2): A copolymer having a repeating structure of a constitutional unit (f1-1-1) and a constitutional unit (f2-3)
Fluororesin (F-3): A copolymer having a repeating structure of a constitutional unit (f1-1-1) and a constitutional unit (f2-4)
Fluororesin (F-4): A copolymer having a repeating structure of a constitutional unit (f1-2-2) and a constitutional unit (f2-1)

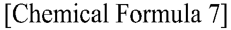

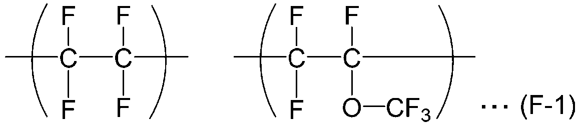

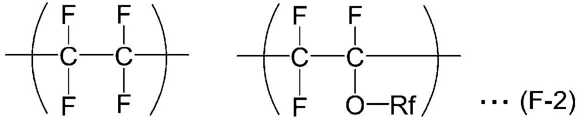

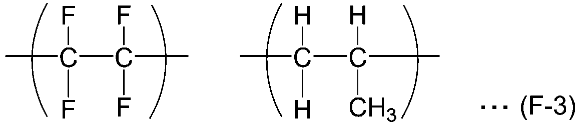

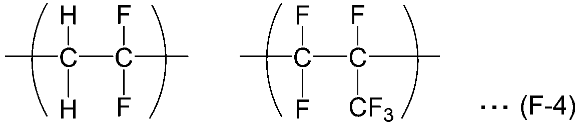

Fluororesin (F-5): A copolymer having a repeating structure of a constitutional unit (f1-2-1), a constitutional unit (f1-1-1), and a constitutional unit (f2-3)
Fluororesin (F-6): A copolymer having a repeating structure of a constitutional unit (f1-2-2), a constitutional unit (f2-1), and a constitutional unit (f1-1-1)

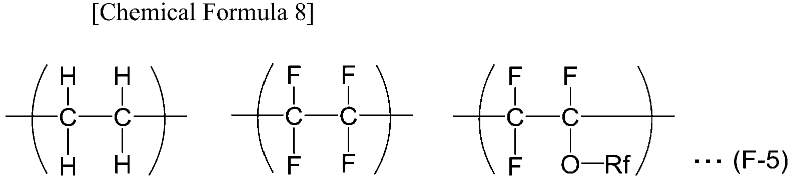

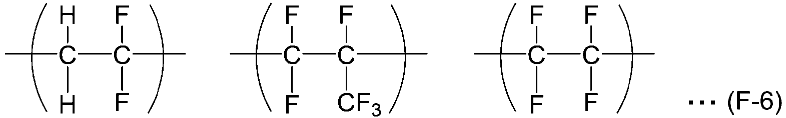

The fluororesin constituting the tubular body 10 preferably contains at least one selected from the group consisting of the fluororesins (F-1) to (F-6), and more preferably contains at least one selected from the group consisting of the fluororesins (F-1) to (F-3).

Examples of the fluororesin constituting the tubular body 10 suitably include a fluororesin (F-3), a fluororesin (F-5), a mixture of a fluororesin (F-1) and a fluororesin (F-2), and a mixture of a fluororesin (F-4) and a fluororesin (F-6).

For the fluororesin constituting the tubular body 10, a produced fluororesin may be used or a commercially available product may be used.

In a case where a produced fluororesin is used, the fluororesin can be synthesized by a publicly known production method. During synthesis, the hardness of the tubular body made of a fluororesin can be adjusted to a desired hardness range by adding carbon black and silica together with the fluorine monomer.
For the fluororesin constituting the tubular body 10, for example, commercially available products (a) to (d) listed below can be used.

(a) A binary copolymer of tetrafluoroethylene and propylene; product name "Fluoro-Power AP", manufactured by Sakura Seal Co., Ltd.
(b) A mixture of a tetrafluoroethylene perfluoromethyl vinyl ether copolymer and a tetrafluoroethylene perfluoroalkyl vinyl ether copolymer; product name "Fluoro-Power FFT", manufactured by Sakura Seal Co., Ltd.
(c) A ethylene-tetrafluoroethylene-perfluoroalkyl vinyl ether ternary copolymer; product name "Viton ETP", manufactured by Sakura Seal Co., Ltd.
(d) A mixture of a vinylidene fluoride hexafluoropropylene binary copolymer and a vinylidene fluoride hexafluoropropylene tetrafluoroethylene ternary copolymer; product name "Ternary Fluororubber", manufactured by Sakura Seal Co., Ltd.

In the tube 100 according to the embodiment described above, the tubular body 10 made of a fluororesin is provided, and thus the drug sorption properties can be decreased in a case where a chemical liquid containing a drug such as a cytosolic substrate is used in a system for feeding.

In addition, the tube 100 is equipped with a tubular body 10 having a thickness of 0.1 to 1 mm and a hardness of 60 to 75. Therefore, it has both flexibility and strength, thereby having favorable moldability into a tube shape and favorable adaptability to pump connection and the like.

In a case of connecting pipes to each other in a liquid feeding device that supplies a chemical liquid to a cell culture chip, the use of the tube 100 for the connection makes it possible to measure the secreted substances from cells with high accuracy. In addition to this, according to the tube 100, the durability of the pump in the cell culture device against squeezing and the like is enhanced, and thus a satisfactory pump function is exhibited. In addition, in a case where the tube 100 is molded, a tube shape having a thickness of 0.1 to 1 mm can be stably molded.

In the present embodiment, the drug includes general drugs referred to as substrate drugs for metabolic enzymes, in addition to components contained in the cytosolic substrate.

### [Other embodiments]

In the tube 100 according to the embodiment described above, the outer layer 20 that coats the outer surface of the tubular body 10 is proved; however, the tube 100 is not limited thereto, and the tube according to the present aspect may consist of only the tubular body. Alternatively, a tube that employs a porous tubular body made of a fluororesin, as a tubular body, may be used.

### (Cell culture device)

The cell culture device according to the second aspect of the present invention is a cell culture device in which the tube according to the first aspect is used for a pipe. The cell culture device according to the present aspect is characterized in that it employs specific tubes for pipe, and various publicly known cell culture devices can be applied to the constitution other than the pipe.

As one embodiment of the cell culture device according to the present aspect, for example, the fluid circuit device or the cell culture device, which is proposed in Patent Document 1 described above, can be applied.

In a cell culture device in which the tube 100 according to the embodiment described above is used for a pipe, the tube 100 can be used for a pump connection tube connectable to a pump, and a linking tube capable of linking the pump connection tube and the relay tube. Therefore, the drug sorption properties are deteriorated as compared with those in the related art, the reliability of cell culture experiments is improved, and it is expected to be applicable to drug efficacy and pharmacological tests, pharmacokinetic tests, safety tests, and the like.

### [Examples]

Hereinafter, the present invention will be described in more detail based on Examples; however, the present invention is not limited to these Examples.

### <Piping tube>

### (Examples 1 to 2 and Comparative Examples 1 to 3)

A tube was molded using the materials shown below and used as a pipe in a fluid circuit device described below.

Silicone resin: Dimethyl silicone; product name "Tigers Polymer SR1154", manufactured by Tigers Polymer Corporation.

Fluororesin (1): A binary copolymer of tetrafluoroethylene and propylene; product name "Fluoro-Power AP", manufactured by Sakura Seal Co., Ltd. The above-described commercially available product (a)

Fluororesin (2): A mixture of a tetrafluoroethylene perfluoromethyl vinyl ether copolymer and a tetrafluoroethylene perfluoroalkyl vinyl ether copolymer; product name "Fluoro-Power FFT", manufactured by Sakura Seal Co., Ltd. The above-described commercially available product (b)

Fluororesin (3): Polytetrafluoroethylene; product name "PTFE", manufactured by Chemours Company.

Fluororesin (4): A binary copolymer of tetrafluoroethylene and propylene; product name "Fluoro-Power APC", manufactured by Sakura Seal Co., Ltd.

### [Tube molding method]

As a die molded product, a tube having an inner diameter of 1 mm, an outer diameter of 2 mm, and a length of 70 mm was molded using the material described above. Each of the tubes obtained by molding is shown below.

The hardness of the tubular body in each tube is a value determined by preparing a plate-shaped test piece (molded body of each resin) using the above-described material according to the above-described procedure (1), preparing a testing machine in the procedure (2), and then measuring the hardness of the plate-shaped test piece (molded body of each resin) according to the procedure (3).

Tube made of silicone resin: hardness of tubular body: 54, tube thickness: 0.50 mm
Tube made of a fluororesin (1): hardness of tubular body: 68, tube thickness: 0.50 mm
Tube made of Fluororesin (2): hardness of tubular body: 75, tube thickness: 0.50 mm
Tube made of Fluororesin (3): hardness of tubular body: 100, tube thickness: 0.50 mm
Tube made of Fluororesin (4): hardness of tubular body:55, molding was not possible.

### <Evaluation>

As a simple model of a cell culture device, a fluid circuit device as shown in FIG. 2 was prepared, and each tube was evaluated.

FIG. 2 is a schematic view of a fluid circuit device that is used in the evaluation.

A fluid circuit device 200 shown in FIG. 2 is equipped with a container 220 that stores a chemical liquid M, a pump drive unit 240, pipes 210 and 230, and a pump tube 250. The pump tube 250 is attached along the pump drive unit 240 so that the power of the pump drive unit 240 (roller rotation) is transmitted.

The pipe 210 is disposed between the container 220 and the pump drive unit 240 and is connected to one end of the pump tube 250. The pipe 230 is disposed between the container 220 and the pump drive unit 240 and is connected to the other end of the pump tube 250.

In the fluid circuit device 200, a vacuum state is generated in the pipe 210 in a case where the pump drive unit 240 is operated (the roller rotates). The chemical liquid M is sucked up from the container 220 into the pipe 210 and flows into the pump tube 250, released from the pump tube 250 into the pipe 230, and then flows to the container 220, thereby being circulated.

For the pipes 210 and 230, a tube made of PEEK was used.

For the pump drive unit 240, RP-6R01S-3P6A-DC10VS-1 (manufactured by TAKASAGO DENKI COGYO CORPORATION) was used.

For the controller, CECS-0100-1 (manufactured by TAKASAGO DENKI COGYO CORPORATION) was used.

For the pump tube 250, each of the above-described tubes (inner diameter: 1 mm, outer diameter: 2 mm; tube length: 70 mm), that is, a silicone resin tube and each of a tube made of a fluororesin (1), a tube made of a fluororesin (2), a tube made of a fluororesin (3), and a tube made of a fluororesin (4) were used.

As the chemical liquid M, a cocktail substrate solution was prepared by mixing 50 uM phenacetin, 5 uM diclofenac, 50 uM S-mephenytoin, 10 uM bufuralol, and 5 uM midazolam.

The circulation of the chemical liquid M was carried out by perfusion by setting a flow rate to 339 uL/hr with a controller.

### [Evaluation of drug sorption properties]

By using each of a chip made of a silicone resin and a tube made of a fluororesin (1), the chemical liquid M was perfused into the pump tube 250 by setting the flow rate to 339 uL/hr.

Before the start of perfusion, and at each of a moment after elapse of 1 hour, a moment after elapse of 2 hours, a moment after elapse of 4 hours, and a moment after elapse of 24 hours after the start of perfusion, 30 µL of the chemical liquid M was collected from container 220, and the concentration of each substrate was measured by LC-MS/MS.

The Initial (0 hr) before the start of perfusion was set to a residual rate of 100%, and the residual rate (average value of N = 3 measurements) of each substrate in the cocktail substrate solution at the moment after elapse of each time after the start of perfusion was determined.

Table 1 shows the results of the residual rate of each substrate in the cocktail substrate solution at a moment after elapse of 24 hours after the start of perfusion.

In addition, Tables 3A and 3B show a graph obtained by plotting each of the residual rates of bufuralol and midazolam in the substrate at a moment after elapse of 1 hour, a moment after elapse of 2 hours, a moment after elapse of 4 hours, and a moment after elapse of 24 hours after the start of perfusion.

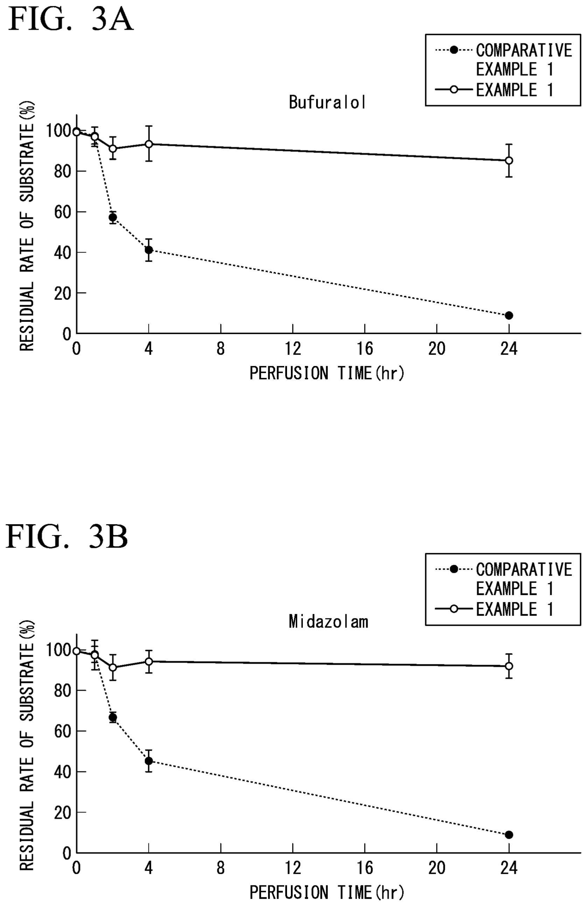

FIG. 3A is a graph showing a change in the residual rate of bufuralol in a cocktail substrate solution versus perfusion time. FIG. 3B is a graph showing a change in the residual rate of midazolam in a cocktail substrate solution versus perfusion time.

**[Table 1]**

| At moment after elapse of 24 hours | Substrate | Comparative Example 1 | Example 1 |
|---|---|---|---|
| | | Tube made of silicone resin (hardness of tubular body: 54) | Tube made of fluororesin (1) (hardness of tubular body: 68) |
| Residual rate of each substrate (%) | Phenacetin | 80.8 | 102.4 |
| | Diclofenac | 94.3 | 100.1 |
| Average value at N=3 | S-Mephenytoin | 81.4 | 102.0 |
| | Bufuralol | 9.2 | 85.6 |
| | Midazolam | 9.1 | 92.5 |

From the results shown in Table 1 at a moment after elapse of 24 hours after the start of perfusion, in all five kinds of substrates, in a case where the tube made of the fluororesin (1) in Example 1 was used, the residual rate was high as compared with a case where a tube made of a silicone resin in Comparative Example 1 was used.

From the behavior of the change in the residual rate at the moment after elapse of each time after the start of perfusion shown in FIG. 3A and FIG. 3B, in a case where the tube made of the fluororesin (1) in Example 1 was used, it can be confirmed that the decrease in the residual rate of the substrates (bufuralol and midazolam) with the lapse of time is almost suppressed, and the residual rate has maintained at 80% or more at a moment after elapse of 24 hours. In a case where the tube made of the silicone resin in Comparative Example 1 was used, it can be confirmed that the residual rate of the substrates (bufuralol and midazolam) has decreased to approximately 50% or less at a moment after elapse of 4 hours after the start of perfusion and has decreased to 10% or less at a moment after elapse of 24 hours.

From the above, it has been confirmed that the tube made of the fluororesin (1) in Example 1 has low drug sorption properties.

### [Evaluation of moldability into tube shape]

A tube-shaped die molded product was molded according to [Tube molding method] described above, the dimensions were measured with a vernier caliper, and the moldability into a tube shape was evaluated according to the following evaluation criteria. The results are shown in Table 2.

### Evaluation criteria

A: Molding could be carried out, and a tube shape could be obtained to have a length of +0.8 mm or less and an outer diameter of +0.2 mm or less from the predetermined dimensions.
B: Molding could be carried out; however, there were errors of more than +0.8 mm in length and more than +0.2 mm in outer diameter from the predetermined dimensions.
C: Molding itself could not be carried out.

### [Evaluation of adaptability to pump connection and the like]

In the same manner as in [Evaluation of drug sorption properties] described above, a liquid feeding experiment, in which the chemical liquid M was perfused into the pump tube 250 by setting the flow rate to 339 uL/hr by using each of chips made of a silicone resin and tubes made of fluororesins (1) to (4), was carried out. Then, the adaptability to pump connection, and the like was evaluated according to the following evaluation criteria. The results are shown in Table 2.

### Evaluation criteria

A: Liquid feeding could be carried out for 24 hours or more as a pump tube without a change in flow rate.
B: A change in flow rate occurred before 24 hours elapsed as a pump tube.
C: It does not function as a pump tube (liquid feeding cannot be carried out).

**[Table 2]**

| | Tube | Hardness of tubular body | Evaluation | |
|---|---|---|---|---|
| | | | Moldability into tube shape | Adaptability to pump connection and the like |
| Example 1 | Tube made of fluororesin (1) | 68 | A | A |
| Example 2 | Tube made of fluororesin (2) | 75 | B | B |
| Comparative Example 1 | Tube made of silicone resin (2) | 54 | B | B |
| Comparative Example 2 | Tube made of fluororesin (3) | 100 | B | C |
| Comparative Example 3 | Tube made of fluororesin (4) | 55 | C | B |

From the results shown in Tables 1 and 2, it could be confirmed that the tubes of Examples 1 and 2 to which the present invention is applied have low drug sorption properties and also have favorable moldability into a tube shape and favorable adaptability to pump connection and the like.

The preferred examples of the present invention have been described as above; however, the present invention is not limited to these Examples. Additions, omissions, substitutions, and other modifications of the configuration can be made without departing from the spirit of the present invention. The present invention is not limited by the description described above and is limited only by the scope of the attached claims.

### REFERENCE SIGNS LIST

10 Tubular body
20 Outer layer
100 Tube
200 Fluid circuit device
210 Pipe
220 Container
230 Pipe
240 Pump drive unit
250 Pump tube

## Claims

1. A piping tube in a cell culture device, comprising:
a tubular body made of a fluororesin,
wherein the tubular body is made of a fluororesin having a constitutional unit (f1-1) represented by General Formula (f1-1) and a constitutional unit (f2) represented by General Formula (f2),
a hardness of the tubular body is 60 to 75, and
a thickness of the tube is 0.1 to 1 mm, 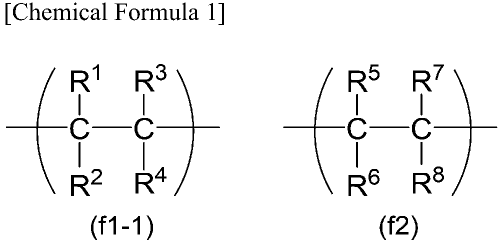
[in the formula, R¹ to R⁷ are each independently a fluorine atom or a hydrogen atom, R⁸ is a trifluoromethyl group, a methyl group, or a group represented by -O-Rf, and Rf is a fluoroalkyl group having 1 to 15 carbon atoms, provided that at least one of R¹ to R⁸ contains a fluorine atom].

2. The tube according to Claim 1, wherein the tubular body is made of a fluororesin having the constitutional unit (f1-1), the constitutional unit (f2), and a constitutional unit (f1-2) represented by General Formula (f1-2) (however, the constitutional unit (f1-2) has a structure different from a structure of the constitutional unit (f1-1)), 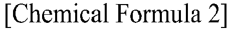 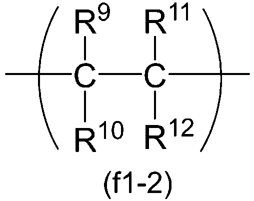 [in the formula, R⁹ to R¹² are each independently a fluorine atom or a hydrogen atom].

3. A cell culture device, wherein the tube according to Claim 1 or 2 is used for a pipe.
